# EUROPÄISCHE PATENTSCHRIFT

(11) **EP 3 554 402 B1**
(45) Veröffentlichungstag und Bekanntmachung des Hinweises auf die Patenterteilung: **26.05.2021**
(21) Anmeldenummer: 17835595.4
(22) Anmeldetag: 11.12.2017
(51) Int. Cl.: A61B 17/80, A61B 17/82

(54) **IMPLANTAT ZUR OSTEOSYNTHESE SOWIE IMPLANTATBAUSATZ MIT IMPLANTAT**
IMPLANT FOR OSTEOSYNTHESIS, AND IMPLANT KIT COMPRISING AN IMPLANT
IMPLANT POUR OSTÉOSYNTHÈSE ET KIT DE MONTAGE D'IMPLANT AVEC IMPLANT

(30) Priorität: 15.12.2016 DE 102016124528
(43) Veröffentlichungstag der Anmeldung: 23.10.2019
(73) Patentinhaber: Karl Leibinger Medizintechnik GmbH & Co. KG, 78570 Mühlheim (DE)
(72) Erfinder: LEIBINGER, Christian, 78570 Mühlheim (DE); KOHLER, Klaus, 78570 Mühlheim (DE)
(74) Vertreter: Winter, Brandl - Partnerschaft mbB
(86) Internationale Anmeldenummer: PCT/EP2017/082221
(87) Internationale Veröffentlichungsnummer: WO 2018/108812

(56) Entgegenhaltungen:
- WO-A1-2015/131106
- GB-A- 2 435 429
- US-A1- 2007 123 880

## Beschreibung

Die Erfindung betrifft ein Implantat zur Osteosynthese / zum Verbinden zweier Knochensegmente eines getrennten / frakturierten Säugerknochens gemäß dem Oberbegriff des Anspruchs 1, mit einem, mehrere Befestigungsmittelaufnahmelöcher aufweisenden Befestigungsbereich sowie zwei sich von dem Befestigungsbereich zumindest abschnittsweise bogenförmig weg erstreckenden Vorsprüngen. Zumindest einer der beiden Vorsprünge ist in Form einer Schnappnase ausgebildet, die auf ein Knochensegment eines Säugerknochens aufschnappbar ist. Das Implantat ist somit alternativ auch als Osteosyntheseimplantat bezeichnet. Auch betrifft die Erfindung einen Implantatbausatz mit einem solchen Implantat sowie mit mehreren Befestigungsmitteln nach Anspruch 10.

Implantate zur Osteosynthese sind aus dem Stand der Technik bereits bekannt. In diesem Zusammenhang offenbart bspw. die DE 10 2008 002 389 B4 eine Vorrichtung zur Osteosynthese sowie zur Fixierung und Stabilisierung, die insbesondere bei Röhrenknochen Anwendung findet. Die Vorrichtung umfasst einen sich in eine erste longitudinale Richtung erstreckenden Trägersteg, wenigstens eine sich lateral vom Trägersteg erstreckende erste Klammer und eine sich lateral vom Trägersteg erstreckende zweite Klammer, wobei die zweite Klammer longitudinal versetzt relativ zur ersten Klammer angeordnet ist. Der Trägersteg weist einen zwischen der ersten Klammer und der zweiten Klammer angeordneten Bereich auf, der in zwei Dimensionen relativ zur longitudinalen Achse des Trägerstegs verbiegbar ausgebildet ist. Auch ist der Trägersteg mit den sich vom Trägersteg weg erstreckenden Klammern einstückig ausgebildet. Ein Verdrehen der ersten Klammer relativ zur zweiten Klammer ist um die longitudinale Achse des Trägerstegs möglich. Insbesondere ist diese plastische Verformung durch eine Dreipunktzange umsetzbar. Weiterer Stand der Technik ist aus der WO 2015/131106 A1 bekannt.

Somit sind aus dem Stand der Technik bereits Implantate bekannt, die Vorsprünge in Form von Klammern aufweisen, wobei die Klammern plastisch verformbar an einem Befestigungsbereich angebracht sind.

Als nachteilig hat es sich jedoch herausgestellt, dass die Handhabung dieser Implantate, nämlich insbesondere das Anbringen des Implantats an den separierten Knochenbereichen relativ aufwändig ist. Hierfür ist mittels eines Werkzeuges, etwa einer Dreipunktzange, die beabsichtigte Form des Implantates voreinzustellen. Dies bedeutet, dass das Implantat nach jedem Biegevorgang immer wieder an den zu verbindenden Knochensegmenten angelegt werden muss, um festzustellen, ob das zurechtgebogene Implantat mit seinen Klammern auch die jeweiligen Knochensegmente sachgemäß umgreift und an diesen anliegt. Wenn dies nicht der Fall ist, muss das Implantat wieder von den Knochenteilen abgehoben werden und erneut zurechtgebogen werden. Somit ist der Aufwand für das Anbringen des Implantates an den jeweiligen Knochensegmenten relativ aufwändig.

Es ist daher die Aufgabe der vorliegenden Erfindung, diese aus dem Stand der Technik bekannten Nachteile zu beheben und insbesondere ein Implantat zur Verfügung zu stellen, das in möglichst wenigen Arbeitsschritten für die jeweilige Osteosynthese-Anwendung angepasst werden soll. Insbesondere soll der Aufwand für die Nachbearbeitung des Implantats im Operationszustand weitestgehend vermieden werden.

Dies wird erfindungsgemäß durch den Gegenstand des Anspruchs 1 gelöst. Das heißt, dass zumindest einer der beiden Vorsprünge relativ zu dem Befestigungsbereich elastisch verformbar ist und im befestigten Zustand des Implantats formschlüssig mit einem Knochensegment verbunden ist. Unter einem Aufschnappen ist insbesondere auch eine temporäre elastische Verformung der Schnappnase zu verstehen, die nur einen geringen Verformungsweg / Federweg mit sich bringt. Insbesondere sind hierbei auch Verformungen zu verstehen, die einen Verformungsweg / Federweg der Schnappnase und / oder des gesamten Implantats von einigen 1/10 mm bewirken. Somit ist etwa ein elastisches Verformen der Schnappnase von ca. 0,1 mm bereits ausreichend, um das Implantat über das entsprechende Knochensegment zu schieben und auf dieses aufschnappen zu lassen. Besonders vorteilhaft sind Verformungswege von kleiner als 0,5 mm, alternativ sind jedoch auch Verformungswege von größer als 0,5 mm umsetzbar. Der Befestigungsbereich ist, zumindest um die Befestigungsmittelaufnahmelöcher herum (ringförmig), hinsichtlich seiner Dicke gegenüber den Vorsprüngen verstärkt. Der Befestigungsbereich ist zudem gitterartig ausgebildet und ein erster Steg des Befestigungsbereiches weist eine erste Gruppe an Befestigungsmittelaufnahmelöchern auf, sowie ein, mit dem ersten Steg fest verbundener, zweiter Steg des Befestigungsbereiches weist eine zweite Gruppe an Befestigungsmittelaufnahmelöchern auf.

Dies hat den technischen Vorteil, dass das Implantat im Bereich der Vorsprünge nicht mehr aufwändig an die dreidimensionale Kontur des frakturierten Säugerknochens angepasst werden muss. Die Vorsprünge passen sich aufgrund ihrer elastischen Beschaffenheit an den jeweiligen Bereich des Knochensegmentes selbständig an und hintergreifen das jeweilige Knochensegment im befestigten Zustand fest. Die Vorsprünge sind hierfür bei der Operation unter einer leichten Vorspannung auf das Knochensegment aufzusetzen. Die natürlich bedingten Toleranzen zwischen Knochensegmenten verschiedener Individuen kann durch die Variabilität der Vorspannung kompensiert werden. Zudem können beim Befestigen des Implantates mit den Knochensegmenten die Befestigungsmittel ausreichend stark angezogen werden, ohne das Implantat dabei unbeabsichtigt zu verformen. Die Aufteilung der Befestigungsmittelaufnahmelöcher auf Stege lässt die Befestigung des Implantats bei der Operation einerseits besonders robust, andererseits besonders vielseitig werden. Das aus der DE 10 2008 002 389 B4 bekannte, im Bereich des Brustbeins eingesetzte Implantat soll beabsichtigt durch seine plastische Verformung am Trägersteg sowie den Klammern an die jeweilige Kontur des Brustbeins sowie der Rippen angeglichen werden, bevor es schließlich mit den Knochensegmenten verbunden wird.

Weitere vorteilhafte Ausführungsformen sind in den Unteransprüchen beansprucht und nachfolgend näher erläutert.

Besonders vorteilhaft ist es, wenn das Implantat als Kondylenimplantat, das auch als Kondylenplatte oder Kondylenschuh bezeichnet ist, eingesetzt ist. In diesem Bereich ist ein Anpassen plastisch zu verformender Implantate besonders aufwändig und das erfindungsgemäße Implantat somit besonders effizient einsetzbar.

Weiterhin ist es von Vorteil, wenn die Vorsprünge (beide) jeweils als Schnappnase ausgestaltet sind und derart an dem Befestigungsbereich angebracht sind, dass sie relativ zueinander (feder-)elastisch (voneinander weg) verbiegbar sind. Unter einem (feder-)elastischen Verbiegen ist ein vorübergehendes Aufbringen einer Biegekraft auf den jeweiligen Vorsprung / die jeweilige Schnappnase zu verstehen, die so niedrig ist, dass der Vorsprung nicht plastisch verformt wird und sich anschließend wieder in die Ausgangsstellung selbsttätig zurückstellt. Dadurch wird die Anbringung des Implantats an den frakturierten Knochensegmenten weiter vereinfacht.

Das Implantat weist gesamtheitlich insbesondere eine derartige Festigkeit und Form auf, dass es nahezu über einen großen Teil der Innenfläche hinweg zum Anlegen an den Knochensegmenten vorbereitet / bestimmt ist.

Weiterhin vorteilhaft ist es, wenn die Vorsprünge jeweils eine Dicke (Materialdicke / Blechdicke) zwischen 0,2 mm und 1,5 mm, vorzugsweise zwischen 0,4 mm und 1,1 mm, weiter bevorzugt zwischen 0,45 mm und 0,8 mm, weiter bevorzugt zwischen 0,4 mm und 0,6 mm, besonders bevorzugt zwischen 0,45 mm und 0,55 mm, noch mehr bevorzugt um 0,5 mm / 0,5 mm aufweisen. Dadurch ist das Implantat so ausgestaltet, dass es einerseits mit einer ausreichenden Festigkeit auf das jeweilige Knochensegment aufgeschnappt werden kann, andererseits das jeweilige Knochensegment durch das Aufschnappen der Vorsprünge auch nicht beschädigt wird.

Um das Implantat mittels der Vorsprünge besonders fest vorzufixieren, ist es zweckmäßig, wenn die Vorsprünge bogenförmig ausgebildet sind und sich vorzugsweise von dem Befestigungsbereich aus um mehr als 90° entlang einer gedachten (in einer Ebene verlaufenden oder helixartig verlaufenden) Bogenlinie weg erstrecken. Dadurch ist ein möglichst weites sowie festes Hintergreifen der Knochensegmente im eingesetzten / aufgesetzten Zustand umgesetzt.

Weiter vorteilhaft ist es, wenn das Implantat (samt der Vorsprünge) aus einem Titanwerkstoff, etwa einer Titan-Aluminium-Legierung bzw. einem Titanal-Werkstoff, weiter bevorzugt aus einem Ti6AI4V (/ ASTM-F136) (vollständig) besteht. Dadurch lässt sich das Implantat mit bereits bewährten Werkstoffen kostengünstig herstellen. Alternativ hierzu ist es jedoch auch von Vorteil, wenn das Implantat (samt der Vorsprünge) aus einem Kunststoff, etwa einem Polyetheretherketon (PEEK), besteht, wobei besonders bevorzugt ein faserverstärkter Kunststoff (aufweisend PEEK) verwendet ist. Prinzipiell sind jedoch auch andere biokompatible Materialien, etwa andere Metalle, einsetzbar.

Von Vorteil ist es in diesem Zusammenhang auch, wenn der Befestigungsbereich (zumindest teilweise) plattenförmig (d.h. sich in einer Ebene erstreckend) und/oder (zumindest teilweise) schalenförmig (d.h. sich entlang einer gekrümmten Fläche erstreckend) ausgeformt ist. Dadurch lässt sich das jeweilige Knochensegment besonders robust umgreifen.

In diesem Zusammenhang ist es weiterhin zweckmäßig, wenn der Befestigungsbereich, zumindest um die Befestigungsmittelaufnahmelöcher herum (ringförmig), eine Dicke zwischen 0,6 mm und 2 mm, weiter bevorzugt zwischen 0,8 mm und 1,6 mm, weiter bevorzugt zwischen 0,9 mm und 1,1 mm, besonders bevorzugt um 1 mm / 1mm aufweist. Dadurch ist das Implantat besonders stabil ausgeformt, andererseits jedoch nicht zu dick, um zu viel Platz einzunehmen.

Ist zumindest ein Befestigungsmittelaufnahmeloch als ein Langloch und / oder zumindest ein Befestigungsmittelaufnahmeloch als ein (kreisrundes) Gewindeloch ausgestaltet, ist das Implantat besonders flexibel und stabil an dem frakturierten Säugerknochen anbringbar.

In diesem Zusammenhang ist es auch zweckmäßig, wenn der erste Steg zumindest abschnittsweise schräg zu dem zweiten Steg verläuft. Der erste Steg erstreckt sich bspw. entlang einer gedachten, gebogenen oder geraden Erstreckungslinie, während sich der zweite Steg entlang einer gedachten, gebogenen oder geraden zweiten Erstreckungslinie erstreckt. Die Befestigungsmittelaufnahmelöcher der jeweiligen Stege sind dann entlang dieser gedachten Erstreckungslinie aneinandergereiht. Dadurch lassen sich die Knochensegmente robust zueinander fixieren.

Weiterhin ist es vorteilhaft, wenn zumindest einer der Stege senkrecht zu seiner Längserstreckung gebogen verläuft / ist. Somit ist das Implantat besonders geschickt an die jeweilige Knochengeometrie anpassbar.

Die Erfindung betrifft zudem einen Implantatbausatz mit einem Implantat nach einer der zuvor beschriebenen Ausführungsformen sowie mehreren Befestigungsmitteln, vorzugsweise Knochenschrauben. Dadurch ist auch ein Implantatbausatz besonders stabil und robust an dem jeweiligen zu behandelnden Knochen anbringbar.

In diesem Zusammenhang ist es auch von Vorteil, wenn die Befestigungsmittel zumindest teilweise als Schrauben mit einem Außendurchmesser / Gewindeaußendurchmesser von 5 mm bis 7 mm, besonders bevorzugt um 6 mm, und somit als 5 mm- bis 7 mm-, vorzugsweise 6 mm-Schrauben ausgebildet sind. Besonders bevorzugt sind Befestigungsmittel, die mit einem Außendurchmesser / Gewindeaußendurchmesser von 1,0 mm bis 3,0 mm ausgebildet sind. Dadurch ist das Implantat besonders robust an dem jeweiligen Knochensegment anbringbar und als Kondylenplatte / -schuh einsetzbar.

Die Erfindung wird nun nachfolgend anhand von Figuren näher erläutert, in welchem Zusammenhang verschiedene Ausführungsbeispiele dargestellt sind.

Es zeigen:
- Fig. 1: eine perspektivische Darstellung eines erfindungsgemäßen Implantats gemäß eines ersten Ausführungsbeispiels, wobei insbesondere die Innenseite des schalenförmigen Implantats sowie dessen Vorsprünge in ihrer Erstreckung erkennbar sind,
- Fig. 2: eine perspektivische Darstellung des Implantats nach Fig. 1 von einer Außenseite, die einen ersten Steg eines Befestigungsbereichs und dessen Verdickung um mehrere Befestigungsmittelaufnahmelöcher herum erkennen lässt,
- Fig. 3: eine perspektivische Darstellung des Implantats der Fign. 1 und 2 von seiner Außenseite, wobei das Implantat gegenüber Fig. 2 um ca. 90° räumlich verdreht dargestellt ist, sodass insbesondere ein zweiter Steg sowie dessen Verdickung an den Befestigungsmittelaufnahmelöchern erkennbar ist,
- Fig. 4: eine Ansicht des Implantats der Fign. 1 bis 3 von oben, sodass ein erster Vorsprung des Implantats in seiner gebogenen Erstreckung besonders gut erkennbar ist,
- Fig. 5: eine perspektivische Darstellung des Implantats der Fign. 1 bis 4, wobei nun ein zweiter Vorsprung in seiner Erstreckung gut erkennbar ist,
- Fig. 6: eine perspektivische Darstellung des Implantates nach den Fign. 1 bis 5 in einem am Säugerknochen eingesetzten Zustand,
- Fig. 7: eine perspektivische Darstellung eines Implantats gemäß eines zweiten Ausführungsbeispiels, wobei das Implantat von einer Außenseite dargestellt ist und, gegenüber dem Implantat des ersten Ausführungsbeispiels, eine durchgängig einheitliche Materialdicke aufweist,
- Fig. 8: eine perspektivische Darstellung des Implantats nach Fig. 7 von einer Innenseite, sodass wiederum die Vorsprünge gut erkennbar sind,
- Fig. 9: eine perspektivische Darstellung eines Implantats gemäß eines dritten Ausführungsbeispiels von einer Außenseite, wobei die beiden Stege des Befestigungsbereiches nun an zwei Enden fest miteinander verbunden sind,

- Fig. 10: eine perspektivische Darstellung des Implantats nach Fig. 9 von einer Innenseite aus, in der wiederum die Vorsprünge gut erkennbar sind, und
- Fig. 11: eine perspektivische Darstellung eines Implantats gemäß eines vierten Ausführungsbeispiels, von einer Außenseite, wobei nur an dem ersten Steg Vorsprünge angeordnet sind.

Die Figuren sind lediglich schematischer Natur und dienen ausschließlich dem Verständnis der Erfindung. Die gleichen Elemente sind mit denselben Bezugszeichen versehen.

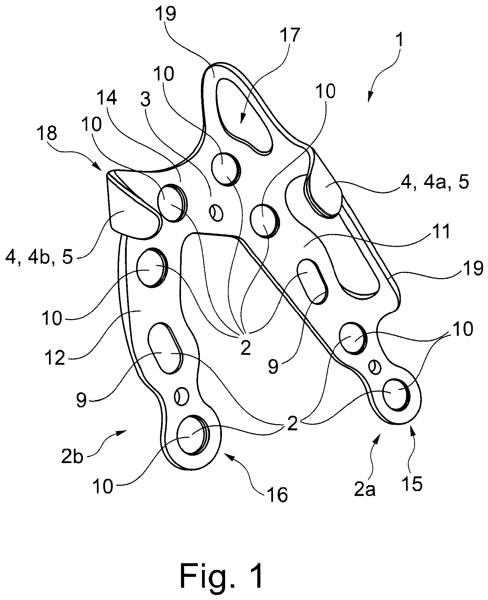

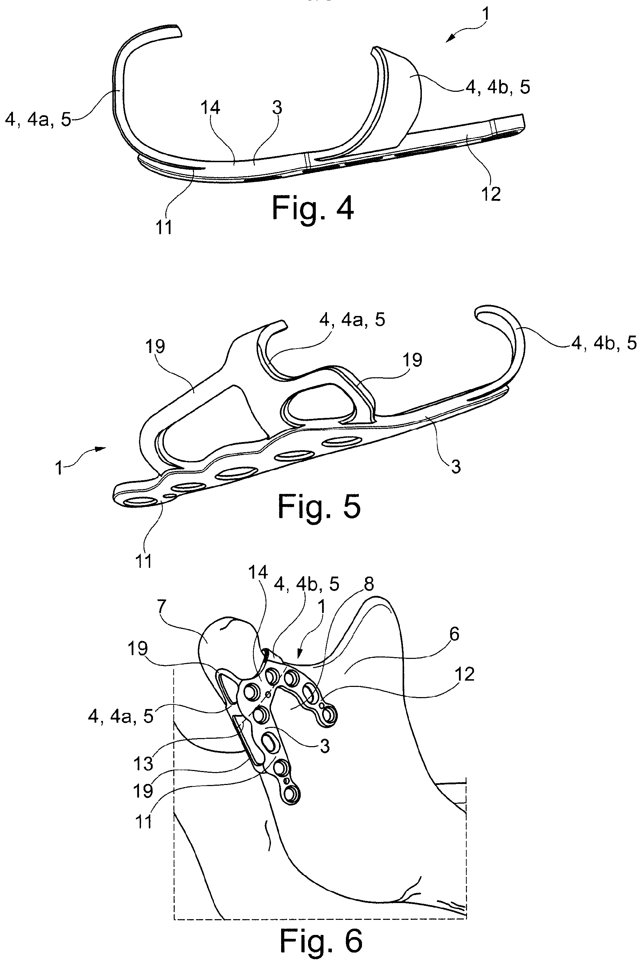

In den Fign. 1 bis 6 ist zunächst ein bevorzugtes erstes Ausführungsbeispiel eines erfindungsgemäßen Implantats 1 anschaulich dargestellt. Wie insbesondere in Fig. 6 gut zu erkennen, dient dieses Implantat 1 auf übliche Weise für Osteosyntheseanwendungen, d.h. zum Verbinden zweier zuvor getrennter / fragmentierter Knochensegmente 7 und 8 eines Säugerknochens 6. Das Implantat 1 ist aufgrund seiner Ausformung, die nachfolgend näher beschrieben wird, besonders für den Einsatz an einem Kollum, hier einem Kiefergelenk, ausgestaltet. In weiteren Ausführungen ist es jedoch auch möglich, dieses Implantat 1 an anderen Bereichen eines Skeletts des Säugers einzusetzen, etwa am Wadenbein oder an Röhrenknochen, wie dem Schlüsselbein. Das Implantat 1 ist in diesem Ausführungsbeispiel somit als Kondylenschuh ausgeführt.

In Fig. 6 ist das Implantat 1 bereits auf den Knochensegmenten 7, 8 des Säugerknochens 6 aufgesetzt, wobei eine theoretische, die beiden Knochensegmente 7, 8 separierende Trennlinie 13 mit einer Strichlinie gekennzeichnet ist. Die Trennlinie 13 teilt den Säugerknochen 6 in das erste Knochensegment 7 sowie das zweite Knochensegment 8 auf. Das Implantat verbindet diese beiden Knochensegmente 7, 8 im befestigten Zustand mittels mehrerer hier der Übersichtlichkeit halber nicht weiter dargestellter Befestigungsmittel, nämlich Knochenschrauben, miteinander. Das Implantat 1 ist so ausgebildet und an die Knochensegmente 7, 8 angepasst, dass es mit seiner den Knochensegmenten 7, 8 zugewandten Innenfläche weitestgehend vollständig an den Knochensegmenten 7, 8 in dem aufgesetzten Zustand anliegt. Es sei darauf hingewiesen, dass auch die Implantate 1 der zweiten bis vierten Ausführungsbeispiele jeweils auf ähnliche Weise wie das Implantat 1 des ersten Ausführungsbeispiels ausgestaltet sowie eingesetzt sind, weshalb der Übersichtlichkeit halber nachfolgend lediglich die wesentlichen Unterschiede zwischen den einzelnen Implantaten 1 näher beschrieben sind.

Wie in Fig. 1 gut zu erkennen, ist das Implantat 1 gesamtheitlich schalenförmig ausgebildet. Das Implantat 1 weist einen Befestigungsbereich 3 auf. Der Befestigungsbereich 3 ist hier im Wesentlichen ebenfalls schalenförmig / leicht gebogen in Gitterstruktur ausgebildet, wie auch in Fig. 4 zu erkennen ist. Der Befestigungsbereich 3 weist mehrere Befestigungsmittelaufnahmelöcher 2 auf, in die jeweils ein Befestigungsmittel in Form einer Knochenschraube im befestigten Zustand eingebracht ist oder eingebracht werden kann. Die Befestigungsmittelaufnahmelöcher 2 sind jeweils als Durchgangsloch ausgebildet.

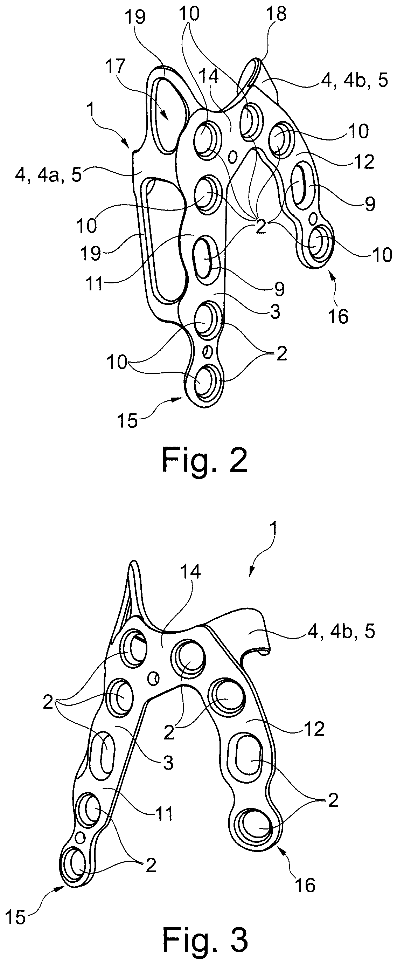

Der Befestigungsbereich 6 weist einen ersten Steg 11 auf. Der erste Steg ist in seiner Längserstreckung besonders gut in Fig. 2 erkennbar. Der erste Steg 11 weist eine gleichbleibende (erste) Dicke auf. Der erste Steg 11 erstreckt sich im Wesentlichen entlang einer gedachten, ersten Erstreckungslinie, die gerade verläuft.

Des Weiteren weist der Befestigungsbereich 6 einen zweiten Steg 12 auf. Der zweite Steg 12 ist wiederum länglich ausgebildet und verläuft, wie in Fig. 3 gut zu erkennen, in seiner Längsrichtung leicht gebogen, d.h. entlang einer gedachten gebogenen, zweiten Erstreckungslinie. Der zweite Steg 12 weist ebenfalls eine gleichbleibende Dicke auf.

Die beiden Stege 11 und 12 des Befestigungsbereichs 6 sind in einem Scheitelbereich 14 einteilig, nämlich stoffeinteilig, miteinander ausgeführt / verbunden. Die beiden Stege 11 und 12 verlaufen im Wesentlichen V- / U-förmig, sodass sich ein Abstand zwischen ihren beiden gedachten Erstreckungslinien mit zunehmender Entfernung zum Scheitelbereich 14 vergrößert.

Ein erstes Ende 15 des ersten Steges 11 in Form eines distalen Endes steht von dem Scheitelbereich 14 aus in die gleiche Richtung ab wie ein erstes Ende 16 des zweiten Steges 12 in Form eines distalen Endes. An ihren beiden den jeweiligen ersten Enden 15, 16 abgewandten zweiten Enden 17 und 18 sind die beiden Stege 11 und 12 miteinander verbunden und bilden den Scheitelbereich 14 aus. Die Stege 11 und 12 sind alternativ auch als Schenkel bezeichnet. Die Stege 11 und 12 sind gleich dick ausgebildet.

Mit dem Befestigungsbereich 3 sind, wie wiederum in Fig. 1 zu erkennen, zwei Vorsprünge 4 verbunden.

Ein erster Vorsprung 4a schließt an den ersten Steg 11 an. Dieser erste Vorsprung 4a ist (Fig. 4) gebogen ausgebildet und erstreckt sich von dem ersten Steg 11 aus um mehr als 90°, nämlich ca. um 140° bis 170°, entlang einer ersten gedachten Bogenlinie von dem Befestigungsbereich 3 weg. Die erste Bogenlinie erstreckt sich im Wesentlichen in einer Ebene.

Der erste Vorsprung 4a ist, wie in Zusammenwirkung der Fign. 1, 2 und 5 auch deutlich zu erkennen, mittels Abstützstegen 19 zusätzlich verstärkt. Diese Abstützstege 19 sind jeweils mit einem Ende mit dem ersten Vorsprung 4a und mit einem anderen Ende an dem Befestigungsbereich 3, nämlich an dem ersten Steg 11, stoffeinteilig angebracht.

Ein zweiter Vorsprung 4b (Fig. 5) ist ebenfalls gebogen ausgebildet. Der zweite Vorsprung 4b erstreckt sich von dem zweiten Steg 12 aus gebogen weg. Dieser zweite Vorsprung 4b erstreckt sich in einem Winkel entlang einer gedachten, zweiten Bogenlinie zwischen 90° und 150° / 170° von dem ersten Steg 11 aus weg. Die zweite Bogenlinie erstreckt sich räumlich im Wesentlichen helixartig.

Die beiden Vorsprünge 4a, 4b bilden zusammen mit dem Befestigungsbereich 3 sowie den Abstützstegen 19 gesamtheitlich eine Schalenstruktur aus. Damit dienen die Vorsprünge 4a, 4b als Hintergriffselemente, die in einem an dem Knochen 6 / den Knochensegmenten 7, 8 aufgesetzten Zustand diesen Knochen 6 / das jeweilige Knochensegment 7, 8 hintergreifen / umgreifen. Das Implantat 1 besteht gesamtheitlich aus einem Material, nämlich einem biokompatiblen Material in Form eines Metalls, bevorzugt einem Titanwerkstoff / einer Titanlegierung, weiter bevorzugt einer Titan-Aluminium-Legierung, wie einem Ti6AI4V.

Das Implantat 1 ist erfindungsgemäß mit seinen Vorsprüngen 4, 4a, 4b als Schnappnasen 5 ausgeführt. Beide Vorsprünge 4a und 4b sind somit elastisch verformbar. Wie in Fig. 6 erkennbar, sind die Vorsprünge 4a und 4b bereits in ihren groben Abmaßen auf die Geometrie des zu behandelnden Säugerknochens 6 abgestimmt. Bei einer Operation werden die beiden Vorsprünge 4a, 4b auf eines der beiden Knochensegmente 7, 8, hier das erste Knochensegment 7, aufgeschnappt. Die Vorsprünge 4a und 4b sind soweit elastisch verformbar, dass sich das Implantat 1 mit den Vorsprüngen 4a und 4b über das erste Knochensegmente 7 aufschieben / aufschnappen lässt.

Die Vorsprünge 4a und 4b sind hinsichtlich ihrer Dicken zur Ausbildung der Schnappnasen 5 ausgebildet. Die Vorsprünge 4a und 4b weisen eine (zweite) Dicke / Materialdicke von 0,5 mm auf. Es sind jedoch in weiteren Ausführungsbeispielen auch andere Dicken für die Vorsprünge 4a und 4b umsetzbar, etwa mehr als 0,5 mm, bspw. 0,8 oder 1 mm. Die (zweite) Dicke der Vorsprünge 4a und 4b ist stets geringer als die (erste Dicke) des Befestigungsbereichs 3 bzw. der Stege 11, 12. Somit sind die Vorsprünge 4a, 4b elastischer / nachgiebiger als der Befestigungsbereich 3.

Um beim Befestigen möglichst stabil die vorgegebene Geometrie des Implantats 1 beizubehalten, ist der Befestigungsbereich 3, d.h. die Stege 11, 12 um die Befestigungsmittelaufnahmelöcher 2 herum gegenüber den Vorsprüngen 4 sowie den Abstützstegen 19 in ihrer Dicke verstärkt. Der Befestigungsbereich 3 / die beiden Stege 11 und 12 weisen hierbei um die Befestigungsmittelaufnahmelöcher 2, d.h. in Ringbereichen um die Befestigungsmittelaufnahmelöcher 2 herum eine (erste) Dicke von ca. 1 mm auf. Der Befestigungsbereich 3 weist durchgängig die gleiche (erste) Dicke auf. Es sind jedoch auch in weiteren Ausführungsbeispielen andere Dicken für den Befestigungsbereich 3 wählbar, bspw. eine Dicke zwischen 0,8 mm und 1,5 mm, wobei stets die (erste) Dicke der Stege 11, 12 größer als die (zweite) Dicke der Vorsprünge 4a, 4b ist.

Des Weiteren weisen beide Stege 11 und 12 jeweils eine bestimmte Gruppe 2a, 2b an Befestigungsmittelaufnahmelöchern 2 auf. Der erste Steg 11 weist fünf Befestigungsmittelaufnahmelöcher 2 auf, die eine erste Gruppe 2a bilden. Der zweite Steg 12 weist eine zweite Gruppe 2b an Befestigungsmittelaufnahmelöchern 2 mit vier Befestigungsmittelaufnahmelöchern 2 auf. Die Gruppen 2a und 2b sind jedoch prinzipiell nicht auf diese Anzahl festgelegt.

Die Befestigungsmittelaufnahmelöcher 2 sind entweder als Langloch 9, als Gewindeloch 10 oder als Durchgangsloch ohne Gewinde ausgeführt. Der erste Steg 11 weist, von dem zweiten Ende 17 aus hin zum ersten Ende 15 betrachtet, zwei Gewindelöcher 10, ein Langloch 9 sowie wiederum zwei Gewindelöcher 10 aneinandergereiht auf. Der zweite Steg 12 weist, wiederum vom Scheitelbereich 14, d.h. vom ersten Ende 16 aus, hin zum zweiten Ende 18 betrachtet, zwei Gewindelöcher 10, ein Langloch 9 sowie ein Gewindeloch 10 aneinandergereiht auf. Die Anordnung dieser Befestigungsmittelaufnahmelöcher 2 sowie deren Ausgestaltungen sind jedoch prinzipiell auf diese Reihenfolge sowie Ausbildung nicht festgelegt. Es ist auch möglich, statt der Langlöcher 9 alternativ ein Gewindeloch 10 und statt der Gewindelöcher 10 ein Langloch 9 anzubringen oder gar auf die Ausbildung von Langlöchern und Gewindelöchern zu verzichten und diese ausschließlich oder teilweise als Durchgangslöcher ohne Gewinde, d.h. Durchgangsbohrungen, auszubilden.

Die schalenförmige Ausbildung des Implantats 1 ist auch besonders gut in Verbindung mit den Fign. 4 bis 6 erkennbar. Der erste Steg 11 ist normal zu seiner Längserstreckung gebogen. Auch wenn der erste Steg 11 in dieser Form leicht gebogen ist, ist der Bereich aus erstem und zweitem Steg 11 und 12 sowie Scheitelbereich alternativ als Platte bezeichnet / ausgeführt. Die Vorsprünge 4, 4a, 4b werden zudem alternativ auch als Klammern / Spangen bezeichnet.

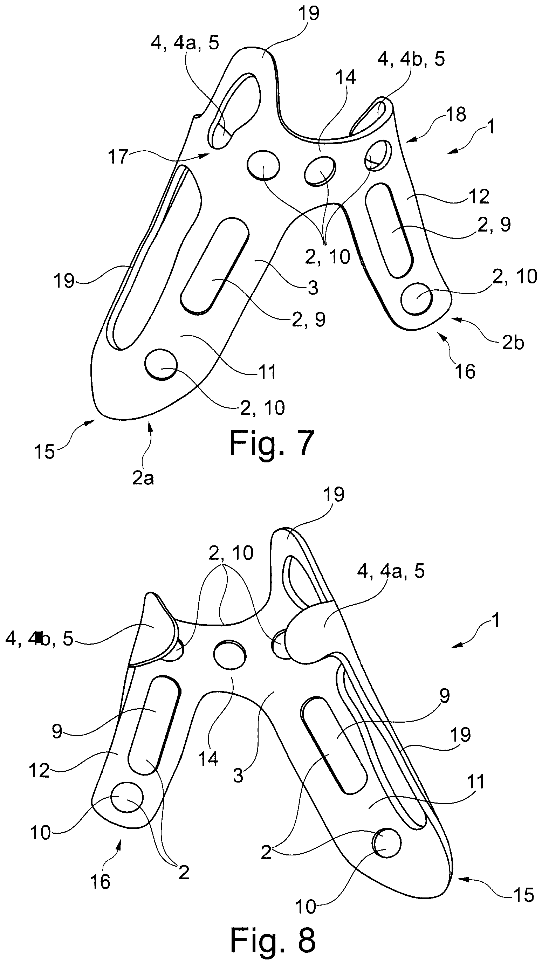

Gemäß dem zweiten Ausführungsbeispiel der Fign. 7 und 8 ist es auch möglich, das Implantat 1 durchgängig mit einer gleichen bestimmten Materialdicke herzustellen. Hierbei ist es besonders bevorzugt, wenn die Materialdicke des gesamten Implantats 1, d.h. sowohl im Befestigungsbereich 3 mit seinen Stegen 11 und 12 als auch an den Vorsprüngen 4, 0,5 mm, in weiteren Ausführungen auch 1 mm beträgt. Hier ist im Scheitelbereich 14 ein zusätzliches Befestigungsmittelaufnahmeloch 2 eingebracht.

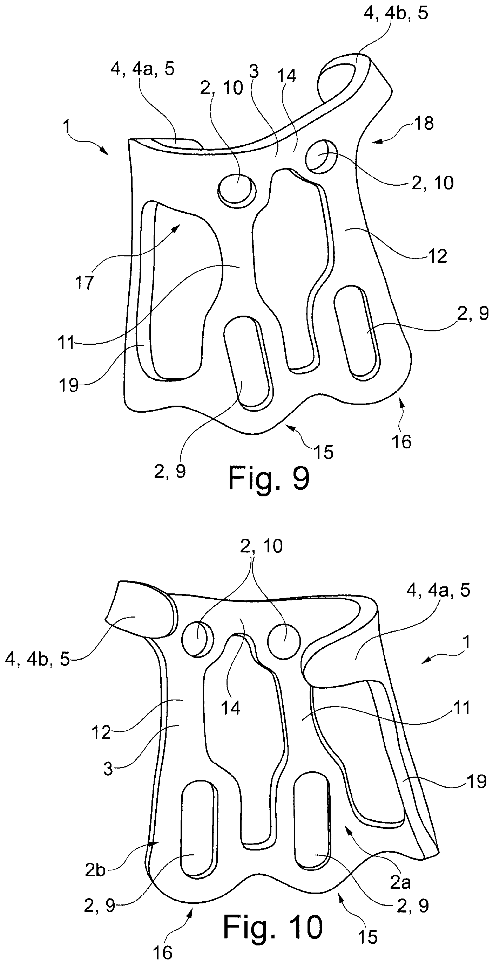

Auch ist in dem weiteren dritten Ausführungsbeispiel gemäß der Fign. 9 und 10 erkennbar, dass der Befestigungsbereich 3 des Implantats 1 nicht auf eine U- / V-förmige Gitterstruktur festgelegt ist, sondern auch alternativ mit einer rechteckförmigen Gitterstruktur ausgebildet werden kann. Somit sind in diesem Ausführungsbeispiel die beiden Stege 11 und 12 sowohl an ihren ersten als auch an ihren zweiten Enden 15 bis 18 jeweils stoffeinteilig miteinander verbunden.

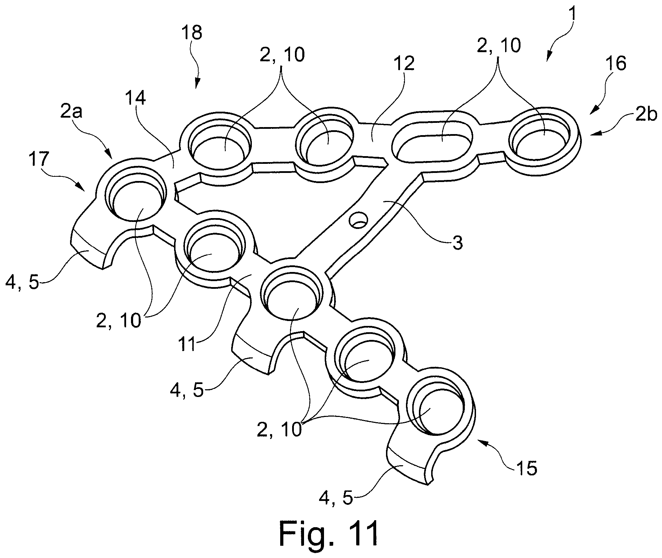

Nach Fig. 11 ist es gemäß einem vierten Ausführungsbeispiel auch möglich auf einen Vorsprung am zweiten Steg zu verzichten und alternativ mehrere Vorsprünge 4, hier drei Vorsprünge 4, an dem ersten Steg 11 anzubringen. Die Vorsprünge 4 erstrecken sich hierbei wiederum direkt von dem ersten Steg 11 aus weg.

In anderen Worten ausgedrückt, ist somit erfindungsgemäß eine Platte (Implantat 1) umgesetzt, die sich extrem einfach und schnell einsetzen lässt. Dies ist insbesondere auf die Manschettenarme (Vorsprünge 4), das flexible Material / die flexible Struktur (bevorzugt 0,5 mm Materialstärke) und die Stützarme (Stege 11, 12) zurückzuführen. Aufgrund dessen bietet diese Platte 1 einen offensichtlichen Vorteil gegenüber bekannten Designs. Aufgrund dessen wird dieses Implantat 1 auch besonders bevorzugt als Kondylenplatte eingesetzt. Alternativ hierzu ist es jedoch auch möglich, die Platte 1 in einer anderen Materialstärke auszubilden, etwa mit einer Materialstärke von 1 mm.

Die Vorgehensweise bei einer Operation ist bevorzugt derart, dass zunächst ein interoraler Zugang zum Kollum geschaffen wird. Daran im Anschluss wird entweder der bereits getrennte Säugerknochen 6 und dessen Knochensegmente 7 und 8 relativ zueinander in die korrekte Stellung vorausgerichtet oder der Säugerknochen 6 bei einer Osteotomie des Kollums mit der Knochensäge zunächst in die beiden Knochensegmente 7 und 8 getrennt. Im Anschluss daran findet eine laterale Reponierung des Fragments / Knochensegments 7 mit einem Werkzeug, vorzugsweise einem Repositionshaken, statt. Im Anschluss daran wird der linke Manschettenarm (erster Vorsprung 4a) der Kondylenplatte 1 kaudal unter dem Fragment / Knochensegment 7 eingehakt. Dann wird die Platte 1 nach kranial gezogen, sodass auch der rechte Manschettenarm (zweiter Vorsprung 4b) kranial einklickt. Im Anschluss daran wird ein erstes Befestigungsmittel, vorzugsweise eine erste Schraube, mit einem Werkzeug, etwa einem Winkelschraubendreher, anterior im Kragen gesetzt. Die zweite Schraube wird ebenfalls im Kragen eingesetzt. Die Fraktur wird reponiert und daran anschließend wird eine dritte Schraube locker mittig in einem Langloch 9 gesetzt, woraufhin die Platte 1 hinsichtlich ihrer Position noch korrigiert werden kann. Daran im Anschluss wird die Schraube im Langloch 9 vollständig fixiert. Zusätzliche Sicherungsschrauben können in die anderen Revisionsmittelaufnahmelöcher weiterhin, je nach Frakturverlauf 13, eingebracht werden.

Somit ist ein Kondylenfragment mit einem einfachen Click-Prinzip zu greifen und zu umschließen. Die beiden Manschettenarme, 4a, 4b umschließen das Fragment optimal, wodurch das Funktionsprinzip, das Fragment "zu fischen" ebenfalls optimal funktioniert. Hierdurch kann das Fragment ohne Probleme reponiert werden. Insbesondere eine Materialstärke der Platte 1 von 0,5 mm, insbesondere im Bereich der Vorsprünge 4a, 4b, ist dabei vorteilhaft. Die Fixierung erfolgt vorzugsweise mit 5 mm Standardschrauben oder 6 mm Standardschrauben. Einige der Schrauben können dabei winkelstabil eingebracht werden. Hierfür weist die Platte 1 teilweise Gewindebohrungen / Gewindelöcher 10 auf, sodass es dem Anwender überlassen wird, die Platte 1 mit Stahlschrauben oder Winkelschrauben anzubringen.

Somit ist auch ein Implantatbausatz, der hier der Übersichtlichkeit halber nicht weiter dargestellt ist, ausgebildet, der das Implantat 1 sowie mehrere Befestigungsmittel in Form von Schrauben, bevorzugt Standardschrauben, aufweist.

### Bezugszeichenliste

- 1: Implantat
- 2: Befestigungsmittelaufnahmeloch
- 2a: erste Gruppe an Befestigungsmittelaufnahmelöchern
- 2b: zweite Gruppe an Befestigungsmittelaufnahmelöchern
- 3: Befestigungsbereich
- 4: Vorsprung
- 4a: erster Vorsprung
- 4b: zweiter Vorsprung
- 5: Schnappnase
- 6: Säugerknochen
- 7: erstes Knochensegment
- 8: zweites Knochensegment
- 9: Langloch
- 10: Gewindeloch
- 11: erster Steg
- 12: zweiter Steg
- 13: Trennlinie
- 14: Scheitelbereich
- 15: erstes (distales) Ende des ersten Steges
- 16: erstes (distales) Ende des zweiten Steges
- 17: zweites Ende des ersten Steges
- 18: zweites Ende des zweiten Steges
- 19: Abstützsteg

## Patentansprüche

1. Implantat (1) zur Osteosynthese, mit einem, mehrere Befestigungsmittelaufnahmelöcher (2) aufweisenden Befestigungsbereich (3) sowie zwei sich von dem Befestigungsbereich (3) zumindest abschnittsweise bogenförmig weg erstreckenden Vorsprüngen (4), wobei zumindest einer der beiden Vorsprünge (4) in Form einer Schnappnase (5) ausgebildet ist, die auf ein Knochensegment (7, 8) eines Säugerknochens (6) aufschnappbar ist, und der Befestigungsbereich (3), zumindest um die Befestigungsmittelaufnahmelöcher (2) herum, hinsichtlich seiner Dicke gegenüber den Vorsprüngen (4) verstärkt ist, **dadurch gekennzeichnet, dass** der Befestigungsbereich (3) gitterartig ausgebildet ist und ein erster Steg (11) des Befestigungsbereiches (3) eine erste Gruppe an Befestigungsmittelaufnahmelöchern (2a) aufweist sowie ein, mit dem ersten Steg (11) fest verbundener, zweiter Steg (12) des Befestigungsbereiches (3) eine zweite Gruppe (2b) an Befestigungsmittelaufnahmelöchern (2) aufweist.

2. Implantat (1) nach Anspruch 1, **dadurch gekennzeichnet, dass** die Vorsprünge (4) jeweils als Schnappnase (5) ausgestaltet sind und derart an dem Befestigungsbereich (3) angebracht sind, dass sie relativ zueinander federelastisch verbiegbar sind.

3. Implantat (1) nach Anspruch 1 oder 2, **dadurch gekennzeichnet, dass** die Vorsprünge (4) jeweils eine Dicke zwischen 0,2 mm und 1,5 mm aufweisen.

4. Implantat (1) nach einem der Ansprüche 1 bis 3, **dadurch gekennzeichnet, dass** die Vorsprünge (4) bogenförmig ausgebildet sind und sich vorzugsweise von dem Befestigungsbereich (3) aus um mehr als 90° entlang einer gedachten Bogenlinie wegerstrecken.

5. Implantat (1) nach einem der Ansprüche 1 bis 4, **dadurch gekennzeichnet, dass** der Befestigungsbereich (3) plattenförmig oder schalenförmig ausgeformt ist.

6. Implantat (1) nach einem der Ansprüche 1 bis 5, **dadurch gekennzeichnet, dass** der Befestigungsbereich (3), zumindest um die Befestigungsmittelaufnahmelöcher (2) herum, eine Dicke zwischen 0,6 mm und 2 mm aufweist.

7. Implantat (1) nach einem der Ansprüche 1 bis 6, **dadurch gekennzeichnet, dass** zumindest ein Befestigungsmittelaufnahmeloch (2) als ein Langloch (9) und/oder zumindest ein Befestigungsmittelaufnahmeloch (2) als ein Gewindeloch (10) ausgestaltet ist.

8. Implantat (1) nach einem der Ansprüche 1 bis 7, **dadurch gekennzeichnet, dass** der erste Steg (11) zumindest abschnittsweise schräg zu dem zweiten Steg (12) verläuft.

9. Implantat (1) nach einem der Ansprüche 1 bis 8, **dadurch gekennzeichnet, dass** zumindest einer der Stege (11, 12) senkrecht zu seiner Längserstreckung gebogen ist.

10. Implantatbausatz mit einem Implantat (1) nach einem der Ansprüche 1 bis 9 sowie mehreren Befestigungsmitteln.

11. Implantatbausatz nach Anspruch 10, **dadurch gekennzeichnet, dass** die Befestigungsmittel zumindest teilweise als Schrauben mit einem Durchmesser von 1,0 mm bis 3,0 mm ausgebildet sind.

## Claims

1. An implant (1) for osteosynthesis, comprising a securing region (3) including a plurality of holes (2) for receiving securing means and two projections (4) which extend away, at least partially curved, from the securing region (3), wherein at least one of the two projections (4) takes the form of a snap-on lug (5) that can snap onto a bone segment (7, 8) of a mammal bone (6), and the securing region (3) is reinforced regarding its thickness, at least around the holes (2) for receiving securing means, as compared to the projections (4), **characterized in that** the securing region (3) is lattice-shaped and a first web (11) of the securing region (3) includes a first group of holes (2) for receiving securing means, and a second web (12) of the securing region (3) which is tightly connected to the first web (11) includes a second group (2b) of holes (2) for receiving securing means.

2. The implant (1) according to claim 1, **characterized in that** the projections (4) are in the form of respective snap-on lugs (5) and are arranged at the securing region (3) so that they are resiliently bendable relative to each other.

3. The implant (1) according to claim 1 or 2, **characterized in that** each of the projections (4) has a thickness ranging from 0.2 mm to 1.5 mm.

4. The implant (1) according to one of the claims 1 to 3, **characterized in that** the projections (4) are formed in curved shape and preferably extend away from the securing region (3) by more than 90° along an imaginary curved line.

5. The implant (1) according to one of the claims 1 to 4, **characterized in that** the securing region (3) is plate-shaped or shell-shaped.

6. The implant (1) according to one of the claims 1 to 5, **characterized in that** the securing region (3) has a thickness ranging from 0.6 mm to 2 mm, at least around the holes (2) for receiving securing means.

7. The implant (1) according to one of the claims 1 to 6, **characterized in that** at least one hole (2) for receiving securing means is configured as a slotted hole (9) and/or at least one hole (2) for receiving securing means is configured as a threaded hole (10).

8. The implant (1) according to one of the claims 1 to 7, **characterized in that** at least portions of the first web (11) extend inclined relative to the second web (12).

9. The implant (1) according to one of the claims 1 to 8, **characterized in that** at least one of the webs (11, 12) is curved perpendicularly to its longitudinal extension.

10. An implant kit comprising an implant (1) according to one of the claims 1 to 9 and comprising a plurality of securing means.

11. The implant kit according to claim 10, **characterized in that** the securing means are at least partly in the form of screws having a diameter ranging from 1.0 mm to 3.0 mm.

## Revendications

1. Implant (1) pour l'ostéosynthèse avec une zone de fixation (3) présentant plusieurs orifices de réception de moyens de fixation (2), ainsi que deux saillies (4) en forme d'arc s'étendant en s'écartant au moins par tronçons de la zone de fixation (3), dans lequel au moins une des deux saillies (4) est conçue sous forme d'un ergot enclipsable (5), qui peut être enclipsé sur un segment osseux (7, 8) d'un os de mammifère (6), et la zone de fixation (3) est renforcée, au moins autour de l'orifice de réception de moyens de fixation (2), concernant son épaisseur par rapport aux saillies (4), **caractérisé en ce que** la zone de fixation (3) est conçue en forme de treillis et une première entretoise (11) de la zone de fixation (3) présente un premier groupe au niveau des orifices de réception de moyens de fixation (2a), ainsi qu'une seconde entretoise (12) de la zone de fixation (3), reliée fixement à la première entretoise (11), présente un second groupe (2b) au niveau des orifices de réception de moyens de fixation (2).

2. Implant (1) selon la revendication 1, **caractérisé en ce que** les saillies (4) sont agencées respectivement comme ergot enclipsable (5) et ainsi sont montées au niveau de la zone de fixation (3), **en ce qu'**elles peuvent être pliées de manière élastique l'une par rapport à l'autre.

3. Implant (1) selon la revendication 1 ou 2, **caractérisé en ce que** les saillies (4) présentent respectivement une épaisseur comprise entre 0,2 mm et 1,5 mm.

4. Implant (1) selon l'une quelconque des revendications 1 à 3, **caractérisé en ce que** les saillies (4) sont conçues en forme d'arc et s'étendent en s'écartant de préférence de la zone de fixation (3) de plus de 90 ° le long d'une ligne arquée fictive.

5. Implant (1) selon l'une quelconque des revendications 1 à 4, **caractérisé en ce que** la zone de fixation (3) est façonnée en forme de plaque ou en forme de coquille.

6. Implant (1) selon l'une quelconque des revendications 1 à 5, **caractérisé en ce que** la zone de fixation (3) présente, au moins autour des orifices de réception de moyens de fixation (2), une épaisseur comprise entre 0,6 mm et 2 mm.

7. Implant (1) selon l'une quelconque des revendications 1 à 6, **caractérisé en ce qu'au** moins un orifice de réception de moyens de fixation (2) est agencé comme un orifice oblong (9) et/ou au moins un orifice de réception de moyens de fixation (2) est agencé comme un orifice fileté (10).

8. Implant (1) selon l'une quelconque des revendications 1 à 7, **caractérisé en ce que** la première entretoise (11) s'étend au moins par tronçons obliquement par rapport à la seconde entretoise (12).

9. Implant (1) selon l'une quelconque des revendications 1 à 8, **caractérisé en ce qu'au** moins l'une des entretoises (11, 12) est courbée perpendiculairement par rapport à son extension longitudinale.

10. Kit d'implant avec un implant (1) selon l'une quelconque des revendications 1 à 9, ainsi que plusieurs moyens de fixation.

11. Kit d'implant selon la revendication 10, **caractérisé en ce que** le moyen de fixation est conçu au moins partiellement sous la forme de vis avec un diamètre allant de 1,0 mm à 3,0 mm.
